# EUROPEAN PATENT APPLICATION

(11) **EP 4 186 538 A1**
(43) Date of publication of application: **31.05.2023**
(21) Application number: 21211079.5
(22) Date of filing: 29.11.2021
(51) Int. Cl.: A61M 1/06, A61N 5/06

(54) **A BREAST PUMP**

(71) Applicant: Koninklijke Philips N.V., 5656 AG Eindhoven (NL)
(72) Inventor: BOURQUIN, Yannyk Parulian Julian, Eindhoven (NL); VARGHESE, Babu, Eindhoven (NL); PALERO, Jonathan Alambra, Eindhoven (NL); BARAGONA, Marco, Eindhoven (NL)
(74) Representative: Philips Intellectual Property & Standards

(57) **Abstract**

A breast pump comprises a breast shield, a stimulator for applying a negative stimulus to promote milk expression, a light source for illuminating the nipple and/or a portion of the breast, and a controller for synchronizing the timing of operation of the light source and the stimulator. The stimulation of blood flow using light has been found to promote milk extraction. In particular, the control of the light source prevents deterioration of the milk quality caused by the illumination.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

Recently, there is a trend towards wearable breast pumps. These devices are generally in a smaller form factor and can be fully worn on the user's body, allowing the user to move around freely. Some of these are small enough to fit into a user's bra. A motor, battery and milk container are for example integrated to form a single unit.

In general, breast pumps are less efficient than babies to extract milk. There is therefore a need to improve the milk expression when using a breast pump.

It has been recognized that light can be used to provide photo therapy, and also to improve milk extraction through the increase in blood flow in the skin. This result from the release of nitric oxide (NO). For example, blue light may be used. Blue light also provides other benefits as for example recognized in US 2018/0289874.

US 2018/0289874 discloses the incorporation of a light source into a breast pump to provide a phototherapy treatment. Blue light is proposed with a wavelength in the range 370nm to 490nm. Red light in the wavelength range 620nm to 700nm is suggested as increasing blood circulation, and near infrared light is suggested as providing pain relief and wound healing.

However, milk should not be exposed to light since such illumination can degrade the quality of the milk. Light sources, such as LEDs suitable for incorporation into a breast pump, are inherently inefficient in terms of the electro-optical conversion. Thus, the management of thermal energy is also an issue.

### SUMMARY OF THE INVENTION

The invention is defined by the claims.

According to examples in accordance with an aspect of the invention, there is provided a breast pump, comprising:
a breast shield for fitting over at least the nipple of a breast thereby to create a cavity over at least the nipple;
a stimulator for applying a stimulus to promote milk expression;
a light source for illuminating the nipple and/or a portion of the breast for enhancing milk expression and/or providing photo therapy treatment; and
a controller for synchronizing the timing of illumination of the nipple and/or portion of the breast by the light source with the stimulus applied by the stimulator.

This breast pump incorporates illumination of the nipple and/or breast, for example to stimulate blood flow. This has been found to promote milk extraction as a result of the increased blood flow in the skin, in particular through the release of nitric oxide. The invention is based on the recognition that the illumination of milk can degrade the quality of the milk, and it provides synchronization between the light source illumination and the stimulus delivered by a stimulator the breast pump. The milk expression takes place at particular times as a result of the stimulus, so the light source illumination can be stopped during at least those times when milk is flowing and could therefore be irradiated by the light source. Thus. milk expression is improved without risking deterioration of the milk quality.

The light source illumination is preferably timed by turning on and off the light source. However, the same result can be achieved by having a constantly actuated light source but operating a shutter or other blocking arrangement to stop the illumination of the nipple and/or portion of the breast.

The light source for example comprises a ring which surrounds the breast including the areola and the nipple, for example an array of LEDs.

The light source is in one example a blue light source with a peak intensity at a wavelength in the range 400nm to 480nm.

Blue light in particular is found to promote increased blood flow. However, blue light is absorbed in milk by riboflavin, and this generates reactive oxygen species. These will reduce the amount of antioxidants in the milk (e.g. vitamin C) and attack polyunsaturated fatty acids.

The wavelength of the peak intensity is for example 453nm +/- 20nm. The light source is for example a blue LED or array of blue LEDs.

Blue light is also known to have other benefits such as for wound healing, and may also have benefits in healing of cracked nipples and mastitis.

The light source for example has an optical flux of 1 to 100 J/cm² and provides an irradiance of 10 to 50 mW/cm².

The light source for example has an output illumination volume, and the controller is adapted to turn off the light source to prevent illumination when there a milk flow within the output illumination volume.

Thus, as mentioned above, the light source is turned off (or blocked) when there is milk flow within the illumination volume of the light source. The light is for illuminating the space around the nipple, and thus illuminates the milk flow when it leaves the nipple. Within the breast, the light is scattered and thus does not reach the milk volumes inside the breast.

The stimulator may be based on the application of a negative pressure to the cavity, but it may instead be a mechanical stimulus, for example applying a vibration or other physical stimulus to the breast tissue and/or nipple to promote milk expression.

For an example in which the stimulator comprises a pressure source for applying a negative pressure, The controller may then be adapted to control the pressure source to provide a cyclic milk expression waveform, and to control the light source cyclically in synchronism with the cyclic expression waveform.

Milk expression then takes places in a pulsed manner, with milk being delivered during the times of deepest vacuum of the cyclic milk expression waveform. The control of the light source is matched to this pulsed milk delivery to prevent illumination during the times when milk is being or has been collected within the illumination volume of the light source, such as during pulses of milk flow.

The controller may be adapted to control the pressure source to provide a milk stimulation waveform in addition to the milk expression waveform, and to turn the light source on for the stimulation waveform.

The light source may be turned on for the full duration of the stimulation phase whereas it is pulsed during the expression phase, in this example. The irradiance level may be different between the stimulation phase and the expression phase, for example higher during the stimulation phase than during the expression phase.

The controller may instead be adapted to control the pressure source to provide a milk stimulation waveform and a cyclic milk expression waveform, and to turn the light source on for the stimulation waveform and off for the cyclic expression waveform.

In this case, the blood flow is increased in advance of the expression phase.

The controller may be adapted to provide pulsed operation of the light source with a first frequency which is lower than a cyclic frequency of a cyclic milk expression waveform, wherein a waveform with a second frequency synchronized with the cyclic milk expression waveform is modulated over the first frequency pulses.

This slow pulsing of the light source (which may be during the stimulation phase and/or the expression phase) is used to provide thermal regulation. It may for example have a period of 5 seconds to 60 seconds. The synchronization with the stimulation is a modulated higher frequency signal over the top of the slow pulses.

For all types of stimulator, the breast pump may comprise a milk sensor for detecting milk flow or presence.

A milk sensor may be used to detect when there is milk present (in the illumination volume of the light source). This may be used as well as using the knowledge that milk is delivered during particular phases of the expression cycle (as explained above), or it may be used instead. Thus, in some examples, the control of the light source is based on feedback sensing, but this will still (indirectly) result in synchronism with the pressure source, and in other cases it may be based on a combination of timing based on the pressure cycle and feedback sensing. The sensing may use an optical sensor, capacitive sensor, impedance sensor, ultrasound sensor or acoustic sensor.

The breast pump may comprise a thermal sensor for sensing a skin temperature, wherein the controller is adapted to switch off the light source to prevent the temperature exceeding a threshold. This prevents excessing heating e.g. of the skin, resulting from the inherent inefficiency of the light source. The threshold is for example a value in the range 45 to 50 degrees.

The breast pump may further comprise a cooling system. This provides another way to prevent excessive heating.

The cooling system may comprise an active cooling system with Peltier elements or an air flow generator, or a passive cooling system.

The light source for example comprises an output window for contact with the skin. This ensures good coupling of the light into the breast tissue.

The invention also provides a method of operating a breast pump for the non-therapeutic expression of milk, comprising:
controlling a light source to illuminate the nipple and/or a portion of the breast for enhancing milk expression and/or providing photo therapy treatment; and
controlling a stimulator to apply a stimulus to promote milk expression,
wherein the method comprises synchronizing the timing of illumination by the light source with the stimulus applied by the stimulator.

The invention also provides a computer program comprising computer program code means which is adapted, when said program is run on processor of the breast pump defined above, to implement the method above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Figure 1: shows a known breast pump system;
- Figure 2: shows a wearable breast pump;
- Figure 3: shows a wearable breast pump modified in accordance with the invention;
- Figure 4: the components of the breast pump of Figure 3;
- Figure 5: shows a pulsed illumination waveform; and
- Figure 6: show the thermal benefit of the pulsed waveform of Figure 5.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump comprising a breast shield, a stimulator for applying a negative stimulus to promote milk expression, a light source for illuminating the nipple and/or a portion of the breast, and a controller for synchronizing the timing of operation of the light source and the stimulator. The stimulation of blood flow using light has been found to promote milk extraction. In particular, the control of the light source prevents deterioration of the milk quality caused by the illumination.

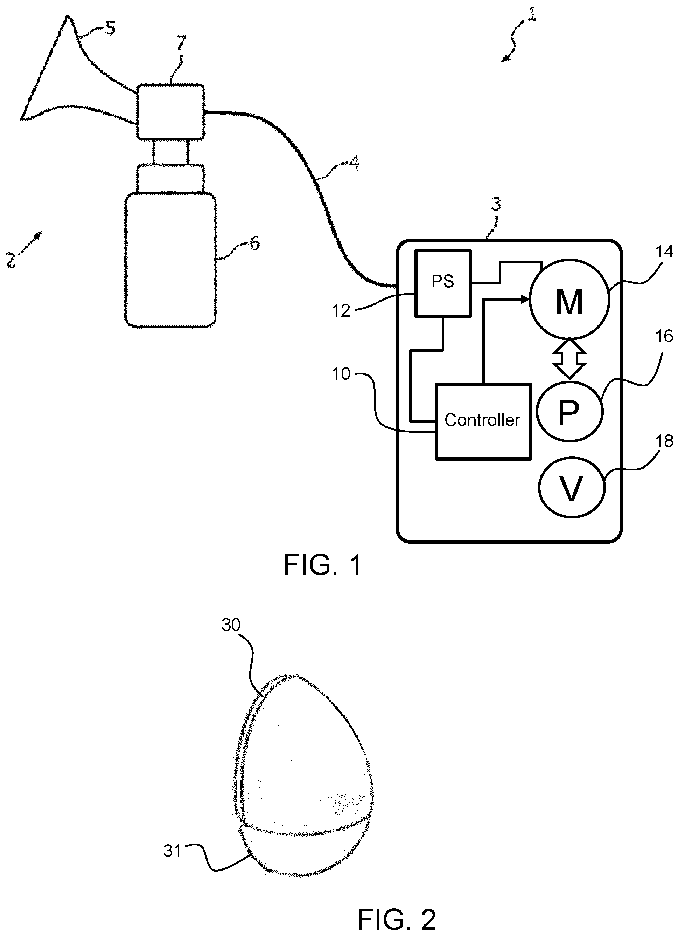

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection container 31.

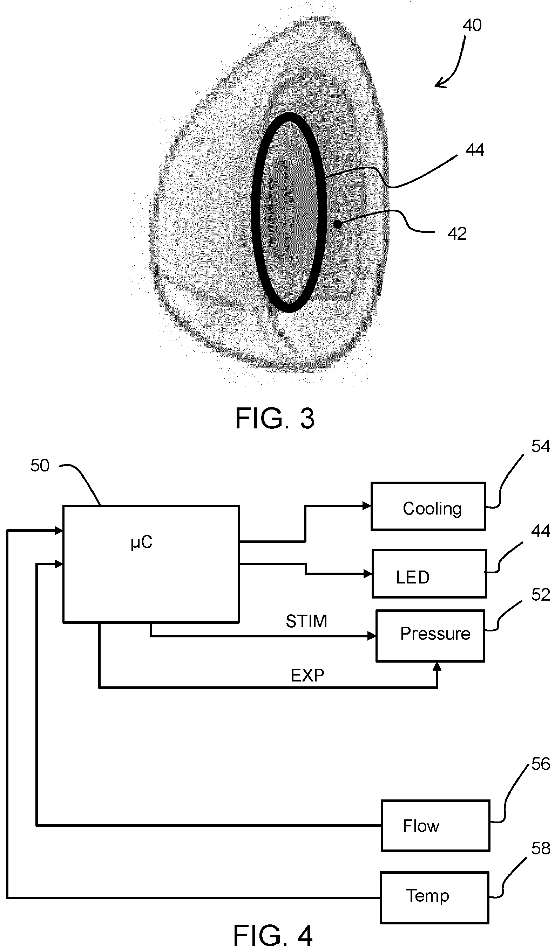

The invention may be applied to a wearable breast pump or a standalone breast pump or indeed any other breast pump configuration. Figure 3 shows wearable breast pump to which the invention has been applied.

The breast pump 40 comprises a breast shield 42 for fitting over at least the nipple of a breast thereby to create a cavity over at least the nipple.

A light source 44 is provided for illuminating the nipple and/or a portion of the breast. In a preferred example, the illumination is for stimulating blood flow in the breast. However, illumination for photo therapy is also known.

Figure 4 shows the internal parts of the breast pump used to control the expression of milk and to control the light source 44.

The light source 44 is controlled by a controller 50. The controller also controls a pressure source 52 for applying a negative pressure to the cavity, in particular a vacuum pump.

The negative pressure is delivered as a cyclic pressure waveform, as mentioned above. The maximum under pressure may be user-adjustable to enable personalization to the user. An expression waveform "EXP" is used to promote milk expression. A stimulation waveform "STIM" may also be provided.

The controller 50 synchronizes the timing of illumination by the light source 44 and the pressure applied by the pressure source. In particular, when the pressure applied results in a flow of milk, the illumination is prevented from reaching the milk flow.

The illumination of the nipple and/or breast is in particular used to stimulate blood flow, which has been found to promote milk extraction through the increased blood flow in the skin, in particular through the release of nitric oxide. The light source illumination is stopped (or blocked) during at least those parts of the pressure cycle when milk is flowing or is collected within the breast shield and could therefore be irradiated by the light source. Thus, milk expression is improved without risking deterioration of the milk quality.

Figure 4 shows additional optional components, which are discussed further below, in particular a cooling system 54, a flow sensor 56 and a temperature sensor 58.

In one preferred example, the light source 44 comprises an array of blue LEDs which are positioned to target the nipple and areola region. When the device is applied on the breast, the blue light may immediately be turned on to increase the blood flow in the breast to enable more optimal milk extraction.

The light source is covered with a transparent window (optically transparent for the wavelength of the radiation of the LEDs), in such a way that the skin contact remains comfortable. The window can be made of glass or other material, for example plastics (PVC, PMMA, Zionex, etc.), transparent ceramics (translucent alumina oxide, etc.) or a soft material such as silicone or other polymer to improve comfort. Contact with the skin provided the best light coupling into skin.

The invention is based on turning off (or shielding/blocking) the light output when there is a milk flow. The presence of milk may be inferred from the pressure applied, in that that there is known relationship between the pressure cycle and a milk flow and milk collection in the breast shield. Thus, actual milk sensing is not needed. However, a milk flow sensor 56 may be provided to enable detection of milk for use as a feedback signal.

Milk presence and flow sensors are well known, for example used for monitoring an amount of expressed milk during breast pumping. Milk presence detection may be based on optical sensors (detecting the passage of a milk droplet), capacitive sensors, impedance sensors (detecting a change in an electric circuit caused by the dielectric permittivity or conductivity of milk), acoustic sensors (for detecting the sound of a milk flow) or ultrasound sensors (for detecting a reflection from a milk droplet).

For the example of an inferred presence of milk, one option is to infer the presence of milk when a user switches from a stimulation mode to an expression mode. However, this will end the illumination during the entire expression period.

An alternative is to synchronize the illumination with the pressure source operation at the level of the individual pump cycles. Milk flows from the nipple when a vacuum is present and the milk accumulates in the breast shield. When the vacuum is released, the milk accumulated in the shield is transferred to the milk container.

Thus there is an absence of milk from a moment slightly after the start of release of vacuum to slightly after the start of the next increase of under pressure (i.e. reduction in pressure). The synchronization may involve providing illumination during this time period when there is an absence of milk, which has been inferred (rather than directly sensed) by the controller.

The most simple way to prevent illumination of milk is to turn off the light source.

Thus, in the first example above when illumination is only during stimulation, the blue source simply is switched off when the breast pump is switched from stimulation to expression. The light treatment would then end as soon as the milk starts flowing.

In another example, if the presence of milk is detected with a sensor, the light source may be turned off when milk is actually present in the breast shield and turned on again when milk is not present. This will indirectly result in synchronization with the pressure source cycle.

In order to generate NO release in the breast tissue, the following light parameters are particularly suitable:
(i) wavelength between 400 nm to 480 nm, preferably 453 nm +/- 20 nm.
(ii) optical energy flux of 1 J/cm² to 100 J/cm².
(iii) irradiance of 10 mW/cm² to 50 mW/cm².
(iv) treatment time of 1 minute to 1 hour.

By way of example, an exposure of 50mW/cm² may be applied over a duration of 100 sec. This is an approximate average time of the stimulation phase of a breast pump. This equates to an energy per unit area of 5 J/cm². This is sufficient to result in NO generation, to enhance the milk expression.

The same irradiance exposure may be maintained during the full session, or the irradiance can be lowered to e.g., 20 mW/cm² during the remaining time of the milk expression session.

In addition to controlling the light source to prevent illumination of the milk, the light source may be controlled to implement thermal management, in particular to remain below thermal safety limits.

Current LED technology allows the manufacture of LEDs with about 30-50% electrical to optical efficiency. Thus, for a 30% efficiency, for each Joule of electrical energy, 0.3 Joules of optical energy and 0.7 Joules of thermal energy are generated. The thermal management LED modules generally is an important issue.

If the thermal energy and optical energy are directed to the skin, the full thermal impact needs to be considered. One approach is to use a temperature sensor 58 as a feedback control sensor, and to regulate the light source current and hence emitted light such that the skin contact temperature remains within desired limits. The allowed temperature increase, within safe limits, can also be used to advantage to stimulate blood flow in addition to the optical effect.

If a heat sinking arrangement is used for the dissipation of heat, the thermal heating as a result of the tissue absorption of the optical energy still needs to be considered. This can be achieved by using numerical models of light absorption and heat distribution in the breast and in the breast pump to identify optimal ranges of operation. Depending on the LED efficiency, a wider or narrower range of possible thermally safe configurations can be designed. Different configurations will have LED arrangements with different optical energy flux and duration of illumination, and will result in different maximum temperature rises due to photothermal effects.

For thermal safety, a maximum surface temperature may be defined, for example in the range 45°C to 50°C, such as 48°C.

Experiments have shown that for optical coupling only, and hence only photothermal heating, thermal safety can be relatively easily achieved for example with an energy delivery in the range 5 J/cm² to 10 J/cm², without requiring active or passive cooling.

When the thermal heating resulting from the poor efficiency of the LEDs is considered, more effective thermal management measures are needed. For example, the energy delivery rate can be reduced but the application time can be increased, to result in the same total energy exposure of the tissue. The higher the electrical to optical efficiency, the shorter the time duration over which the illumination may need to be applied to deliver a required optical energy dose.

The range of safe configurations for thermal management increases when a cooling system 54 is used.

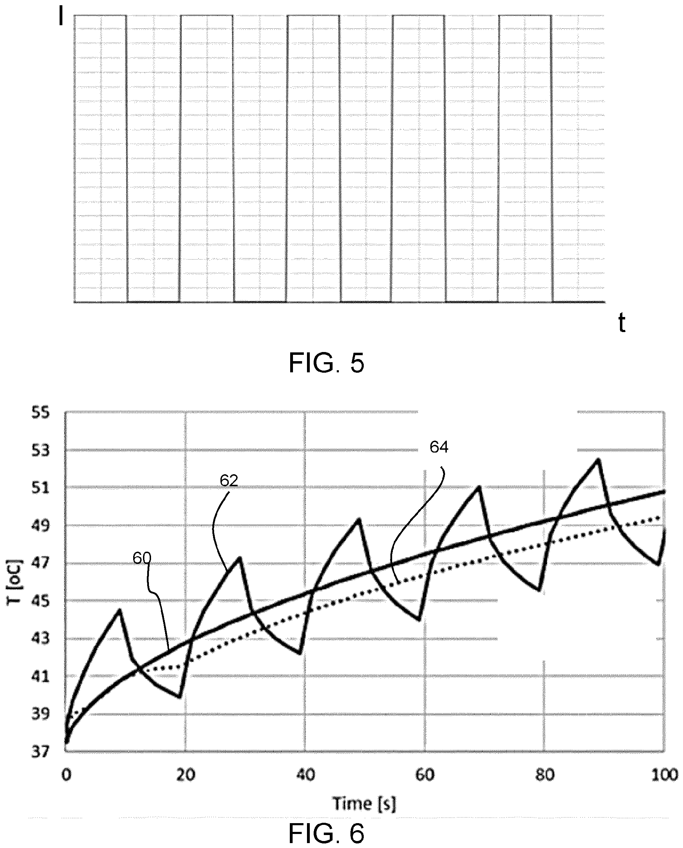

Figure 5 shows another approach for reducing heating. It shows a plot of light source output intensity (I, y-axis) against time (t, x-axis).

A pulsed operation of the light source is provided. The frequency is lower than a cyclic frequency of a cyclic milk expression waveform. For example, the period in Figure 5 may be 20 seconds since it relates to thermal heating and cooling, which is a slower process that the frequency of the expression or stimulation cycles (periods of around 0.5s to Is).

Thus, the higher frequency modulation of the light source, e.g., during the expression phase, may be modulated over the slower waveform shown in Figure 5, or the waveform shown in Figure 5 may be applied during the stimulation phase.

An active or passive cooling system may then be used to reduce the temperature during the non-illumination phases.

Figure 6 shows a plot 60 of skin temperature over time without pulsation and a plot 62 with pulsation. Passive cooling, for example simply due to contact of the skin with the optical window of the light source, is assumed between each pulse.

Plot 64 is a moving average of the plot 62 showing the decrease in average temperature. Even with a basic passive cooling approach, a decrease of approximately 2 degrees in the average temperature is achieved (but with higher temperature peaks).

The cooling system may comprise active cooling during the pauses in the pulses or continuously, for example with Peltier elements, or using a forced air flow. Passive cooling may be applied using fins (with or without a pulsation scheme). Rather than pulsing all LEDs off, the LED illumination pattern over the surface of the breast shield may change over time, for example using successive activation of LEDs rings along the breast shield.

The light source illumination is preferably timed by turning on and off the light source as shown. However, the same result can be achieved by having a constantly actuated light source but operating a shutter or other blocking arrangement to stop the illumination of the nipple and/or portion of the breast.

The light source is preferably implemented using LEDs, but other light sources may be used such as lasers, laser diodes, OLED sheets and electroluminescent emitters.

One illumination of particular interest is blue light, as explained above. However, the same approach may be applied to any light treatment with any desired wavelength, if it is of interest to protect the expressed milk from the illumination.

In the example above, the light source is turned on and off to synchronize the timing of illumination of the nipple and/or portion of the breast with the stimulus applied by the stimulator. However, the light source could be turned on constantly, and a light blocking arrangement (such as an electronic shutter) could be turned on and off to control when the illumination from the light source reaches the nipple and/or breast, and when it is blocked.

Alternatively, a movable light blocking arrangement could be used, For example, a movable light blocking arrangement could be actuated by an applied pressure, so that the synchronization with a pressure waveform is automatically implemented. In such a case, a stimulator in the form of a pressure source is controlled, and this automatically provides synchronization of the illumination that reaches the nipple and/or breast.

The example above uses a pressure waveform, applied to a cavity around the breast. to perform the stimulation and expression functions. However, other forms of stimulus may be used to promote the milk ejection reflex and hence stimulate milk expression.

For example, a stimulation mode may comprise a mechanical stimulation mode by which mechanical pressure is applied to the breast (i.e. physical contact with the breast applying a force to the breast). The milk ejection reflex can be stimulated by this physical interaction with the breast instead of using the ambient pressure of a cavity around the breast.

The invention may be applied to any stimulus which can control the expression of milk at predictable times, so that the illumination can be controlled in a synchronized manner.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump (40), comprising:
a breast shield (42) for fitting over at least the nipple of a breast thereby to create a cavity (42) over at least the nipple;
a stimulator for applying a stimulus to promote milk expression;
a light source (44) for illuminating the nipple and/or a portion of the breast for enhancing milk expression and/or providing photo therapy treatment; and
a controller (50) for synchronizing the timing of illumination of the nipple and/or portion of the breast by the light source with the stimulus applied by the stimulator.

2. The breast pump of claim 1, wherein the light source (44) is a blue light source with a peak intensity at a wavelength in the range 400nm to 480nm.

3. The breast pump of any one of claims 1 to 2, wherein the light source (44) has an optical flux of 1 to 100 J/cm² and provides an irradiance of 10 to 50 mW/cm².

4. The breast pump of any one of claims 1 to 3, wherein the light source (44) has an output illumination volume, and the controller is adapted to turn off the light source to prevent illumination when there a milk flow within the output illumination volume.

5. The breast pump of any one of claims 1 to 4, wherein the stimulator comprises a pressure source (52) for applying a negative pressure to promote milk expression.

6. The breast pump of claim 5, wherein the controller (50) is adapted to control the pressure source (52) to provide a cyclic milk expression waveform, and to control the light source cyclically in synchronism with the cyclic expression waveform.

7. The breast pump of claim 6, wherein the controller (50) is adapted to control the pressure source to provide a milk stimulation waveform, and to turn the light source on for the stimulation waveform.

8. The breast pump of claim 5, wherein the controller (50) is adapted to control the pressure source to provide a milk stimulation waveform and a cyclic milk expression waveform, and to turn the light source on for the stimulation waveform and off for the cyclic expression waveform.

9. The breast pump of any one of claims 5 to 8, wherein the controller (50) is adapted to provide pulsed operation of the light source with a first frequency which is lower than a cyclic frequency of a cyclic milk expression waveform, wherein a waveform synchronized with the cyclic milk expression waveform is modulated over the first frequency pulses.

10. The breast pump of any one of claims 1 to 9, comprising a milk sensor (56) for detecting milk flow or presence.

11. The breast pump of any one of claims 1 to 10, comprising a thermal sensor (58) for sensing a skin temperature, wherein the controller is adapted to switch off the light source to prevent the temperature exceeding a threshold.

12. The breast pump of any one of claims 1 to 11, further comprising a cooling system (54), wherein the cooling system comprises an active cooling system with Peltier elements or an air flow generator, or a passive cooling system.

13. The breast pump of any one of claims 1 to 12, wherein the light source comprises an output window for contact with the skin.

14. A method of operating a breast pump for the non-therapeutic expression of milk, comprising:
controlling a light source to illuminate the nipple and/or a portion of the breast for stimulating blood flow in the breast; and
controlling a stimulator to apply a stimulus to promote milk expression,
wherein the method comprises synchronizing the timing of illumination of the nipple and/or portion of the breast by the light source with the stimulus applied by the stimulator.

15. A computer program comprising computer program code means which is adapted, when said program is run on processor of the breast pump of any one of claims 1 to 13, to implement the method of claim 14.
